**Europäisches Patentamt**

**European Patent Office**

**Office européen des brevets**

(19)

(11) Veröffentlichungsnummer: **0 268 191**
**B1**

(12) # EUROPÄISCHE PATENTSCHRIFT

(45) Veröffentlichungstag der Patentschrift:
**19.09.90**

(51) Int. Cl.⁵: **A61M 5/28**, A61M 5/20

(21) Anmeldenummer: **87116619.5**

(22) Anmeldetag: **11.11.87**

(54) **Injektionsgerät.**

(30) Priorität: **14.11.86 DE 3638984**

(43) Veröffentlichungstag der Anmeldung:
**25.05.88 Patentblatt 88/21**

(45) Bekanntmachung des Hinweises auf die Patenterteilung:
**19.09.90 Patentblatt 90/38**

(84) Benannte Vertragsstaaten:
**AT CH DE FR GB IT LI NL SE**

(56) Entgegenhaltungen:
**EP-A-00 585 36**
**WO-A-85/03446**
**DE-A- 2 812 729**
**DE-A- 3 527 066**
**DE-A- 3 604 826**
**FR-A- 2 350 848**
**US-A-41 945 05**

(73) Patentinhaber: **WILHELM HASELMEIER GMBH & CO.,**
**Vaihinger Strasse 48, D-7000 Stuttgart 80(DE)**

(72) Erfinder: **Gabriel, Jochen, Dipl.-Kaufmann, Im**
**Falkenrain 1, D-7000 Stuttgart 1(DE)**
Erfinder: **Bechtold, Herbert, Dipl.-Ing., Breslauer**
**Strasse 19, D-7031 Ehningen(DE)**
Erfinder: **Hambrecht, Gerhard, Lammstrasse 3,**
**D-6964 Rosenberg-Sindolzheim(DE)**
Erfinder: **Nothdurft, Klaus, Vaihinger Strasse 50,**
**D-7000 Stuttgart 80(DE)**

(74) Vertreter: **Raible, Hans, Dipl.-Ing., Schoderstrasse 10,**
**D-7000 Stuttgart 1(DE)**

ACTORUM AG

## Beschreibung

Die Erfindung betrifft ein Injektionsgerät nach dem Oberbegriff des Patentsnspruchs 1.

Ein Injektionsgerät nach dem Oberbegriff ist bekannt aus der US-A 4 194 505. Dieses Gerät ist ausgebildet zur Aufnahme spezieller Kartuschen, die eine bestimmte Menge Insulin enthalten, beispielsweise 20 Einheiten. Benötigt der Patient nur 17 Einheiten, so hat er die Möglichkeit, am Gerät eine Einstellung vorzunehmen. Dazu stellt er zunächst die Maximaldosis ein, welche einer Nullinie auf der Skala des Geräts entspricht.

Ausgehend hiervon stellt er auf dieser Skala drei Striche ein, entsprechend den drei Einheiten, die er nicht injizieren möchte. Dadurch wird am Gehäuse ein beweglicher Anschlag verstellt, der die Bewegung eines Stößels begrenzt und daher praktisch die wirksame Länge dieses Stößels verkürzt. Anschließend löst der Patient die Injektion aus, wobei zunächst die Injektionsnadel eingestochen und dann die gewünschte Insulindosis aus der Kartusche injiziert wird. – Dieses Gerät ist kompliziert zu bedienen: Man benötigt hierfür Kartuschen mit unterschiedlicher Dosierung, nämlich solche mit 10, 20, 30, 40, 50 bzw. 60 Einheiten Insulin. Ist der Patient blind, so kann er solche Kartuschen leicht verwechseln, und es besteht dann die Gefahr, daß er sich zu viel Insulin spritzt. Auch sind die Rechenoperationen bei der Bedienung dieses Geräts nicht gerade einfach und sinnfällig und können manche Patienten überfordern.

Bei der sogenannten intensivierten Insulintherapie muß nämlich der Patient nach jeder Mahlzeit eine unterschiedliche Menge Insulin spritzen, und es kann dann zu Rechenfehlern kommen. Benötigt der Patient z.B. nach dem Frühstück 8 Einheiten, nach dem Mittagessen 15 Einheiten und nach dem Abendessen 12 Einheiten, so muß er zum Frühstück eine Kartusche für 10 Einheiten verwenden und das Gerät auf minus 2 Einheiten einstellen. Zum Mittag- und Abendessen muß er eine Kartusche mit 20 Einheiten verwenden und dabei das Gerät mittags auf minus 5 Einheiten und abends auf minus 8 Einheiten einstellen. All das erfordert viel Sorgfalt und kann leicht zu Fehlern führen.

Aus der DE-A 2 812 729 kennt man ein Injektionsgerät, das mit einem speziellen Halter für eine Injektionsspritze versehen ist, der durch eine Feder gespannt werden kann. Wird diese Feder ausgelöst, so bewegt sich der Halter in proximaler Richtung, und die Injektionsnadel wird in den Patienten eingestochen.

Zum Auslösen dieser Feder dient ein beweglicher Handgriff, der bei Betätigung zwei Zahnstangen im Gegensinn verschiebt. Eine der Zahnstangen löst bei Verschiebung zunächst die Feder aus, so daß die Nadel in den Patienten eingestochen wird. Anschließend bringt sie bei weiterer Verschiebung einen Stößel zum Anschlag gegen den Kolben der Injektionsspritze, so daß das auf diese zuvor aufgezogene Medikament in den Patienten injiziert wird. Die Lage dieses Stößels relativ zur Zahnstange ist veränderbar, doch hat dies keinen Einfluß auf die Injektionsmenge; diese wird vielmehr nur von der zuvor auf die Spritze aufgezogenen Menge des Medikaments bestimmt, die beim Injektionsvorgang vollständig injiziert wird.

Schließlich kennt man aus der WO 82/02622 (= EP-A 0 058 536, ein Injektionsgerät, mit dem ein Patient aus einer Kartusche mit Insulin mehrere Dosen injizieren kann. Er stellt dazu durch Verdrehen eines Vorstellrades die gewünschte Insulindosis ein, sticht dann die Nadel ein, und dreht anschließend das Voreinstellrad in dessen Nullstellung zurück. Dadurch wird ein Gewindeteil aus einer Hülse herausgeschraubt, drückt gegen den Kolben in der Kartusche, und preßt Insulin aus der Kartusche durch die Injektionsnadel in den Patienten. Nachteilig hierbei ist aber, daß dieses Gerät nur auf der Vorderseite des Körpers verwendet werden kann, während man bei der sogenannten intensivierten Insulintherapie in der Praxis gezwungen ist, auch Injektionen auf der Rückseite des Körpers vorzunehmen, um Hämatome und Hornhautbildung durch zu häufiges Injizieren an derselben Stelle zu vermeiden. Auch ist eine Injektion durch Drehung eines Vorstellrades unter Umständen recht schmerzhaft. Bekanntlich unterscheiden sich die Menschen in ihrer Schmerzempfindlichkeit ganz außerordentlich voneinander: Während manche fast gar keinen Schmerz empfinden und Injektionen klaglos vertragen, sind andere Patienten extrem schmerzempfindlich, besonders die sogenannten Hochhistaminpatienten, und bei letzteren ist diese Art der Injektion nicht sehr beliebt und führt dann dazu, daß die Behandlung vernachlässigt oder sogar ganz unterlassen wird.

Geräte wie diejenigen nach der DE-A 2 812 729 werden in der Branche gewöhnlich als Halbautomaten bezeichnet, während Geräte wie die nach der US-A 4 194 505 gewöhnlich als Vollautomaten bezeichnet werden, da bei ihnen sowohl das Einstechen der Nadel wie die Injektion des Medikaments nach Auslösung durch den Benutzer vollautomatisch ablaufen. Vollautomaten haben den Vorteil, daß mit ihnen auch an der Rückseite des Körpers injiziert werden kann.

Aufgabe der Erfindung ist es, einen verbesserten Vollautomaten zu schaffen, der mehrere Injektionen aus einer Kartusche ermöglicht.

Diese Aufgabe wird nach der Erfindung bei einem eingangs genannten Injektionsgerät gelöst durch die im kennzeichnenden Teil des Anspruchs 1 angegebenen Maßnahmen. Man erhält hierdurch eine sehr einfache, stufenlose Verstellung der Gesamtlänge von Stößel und Verlängerungsanordnung nur durch Drehung der - von außen zugänglichen Verlängerungsanordnung. Bei der Erfindung handelt es sich also um einen sogenannten Vollautomaten nach der obigen Definition. Ein solches Injektionsgerät erlaubt die Aufnahme eines Behälters, der Flüssigkeit, z.B. Insulin, für mehrere Injektionen enthält. Mit Hilfe des Stellglieds kann der Patient für jede Injektion die richtige Dosis einstellen, wobei er den Behälter mit der zu injizierenden Flüssigkeit immer erst nach einiger Zeit zu wechseln braucht, z.B. nach zwei oder drei Tagen. Dabei kann sich der Patient mit diesem Gerät auch an schwer zugänglichen Stellen des Körpers, z.B. der Nierengegend oder dem Oberarm, spritzen, so daß sich dieses Gerät besonders für die sogenannte intensivierte insulintherapie eignet.

Ein Sicherheitsgewinn für den Patienten ergibt sich ferner aus ·folgendem: Die Insulinbehälter ("Kartuschen") verschiedener Hersteller haben z.T. unterschiedliche Größen. Dies birgt das Risiko in sich, daß bei Verwendung einer falschen Kartusche Fehldosierunsen entstehen. Mit der Erfindung kann dies dadurch vermieden werden, daß Form und Kalibrierung des Geräts exakt an die unterschiedlichen Kartuschen unterschiedlicher Hersteller angepaßt werden. Dadurch werden Verwechslungen sicher vermieden.

Eine sehr vorteilhafte, elegante und erfinderische Art der nicht verdrehbaren Führung des Stößels ist in Anspruch 2 angegeben, wobei die im Anspruch 3 angegebene Lösung eine bevorzugte Weiterbildung darstellt.

Das Führungsglied kann eine weitere Funktion übernehmen, wenn es gemäß Anspruch 4 ausgebildet ist, da es dann auch zur Übertragung axialer Kräfte auf die Kombination von Stößel und Verlängerungsanordnung dienen kann.

Für die Verbindung zwischen Stellglied und Verlängerungsanordnung ergeben sich verschiedene vorteilhafte Möglichkeiten, wie sie in den Ansprüchen 6 und 8 angegeben sind. Gemäß Anspruch 7 kann in besonders vorteilhafter Weise der Handgriff gleichzeitig zum Spannen und auch für erforderliche Einstellvorgänge verwendet werden.

Die Auslösevorrichtung wird bevorzugt gemäß Anspruch 10 ausgebildet; der hierzu bevorzugt verwendete Clip kann auch zur Befestigung des Geräts an einer Tasche, einem Kleidungsstück etc. dienen.

Als besonders vorteilhaft erweist sich dabei die Ausführungsform nach Anspruch 11, da hierbei das Führungsglied auch noch eine wichtige Rolle im Auslösemechanismus des Injektionsgeräts übernehmen kann. Dies reduziert die Zahl der Teile und macht hierdurch die Herstellung des Geräts wirtschaftlicher.

Weitere Einzelheiten und vorteilhafte Weiterbildungen der Erfindung ergeben sich aus dem im folgenden beschriebenen und in der Zeichnung dargestellten, in keiner Weise als Einschränkung der Erfindung zu verstehenden Ausführungsbeispiel, sowie aus den übrigen Unteransprüchen. Es zeigt:

Fig. 1 einen Längsschnitt durch ein bevorzugtes Ausführungsbeispiel eines erfindungsgemäßen Injektionsgeräts, im zusammengeschobenen Ruhezustand, und in vergrößertem Maßstab,

Fig. 2 eine teilweise Darstellung des Injektionsgeräts der Fig. 1 im auseinandergezogenen, gespannten Zustand, ebenfalls im vergrößerten Maßstab,

Fig. 3 eine Explosionsdarstellung desjenigen Teiles B des Injektionsgeräts, das für die Aufnahme des Behälters (Kartusche) mit der zu injizierenden Flüssigkeit dient,

Fig. 4 eine Explosionsdarstellung desjenigen Teiles A des Injektionsgeräts, das für die Einstellung (Dosierung) und Durchführung der Injektion dient,

Fig. 5 eine teilweise, raumbildliche Darstellung des Stößels, der Verlängerungsanordnung und des an diesen beiden Teilen befestigten Führungsgliedes; der Stößel hat ein Rechteckgewinde mit z.B. 8 mm Steigung, und

Fig. 6 eine raumbildlich gezeichnete Draufsicht auf ein bevorzugtes Ausführungsbeispiel der Stellvorrichtung für die Dosierung der zu injizierenden Flüssigkeitsmenge.

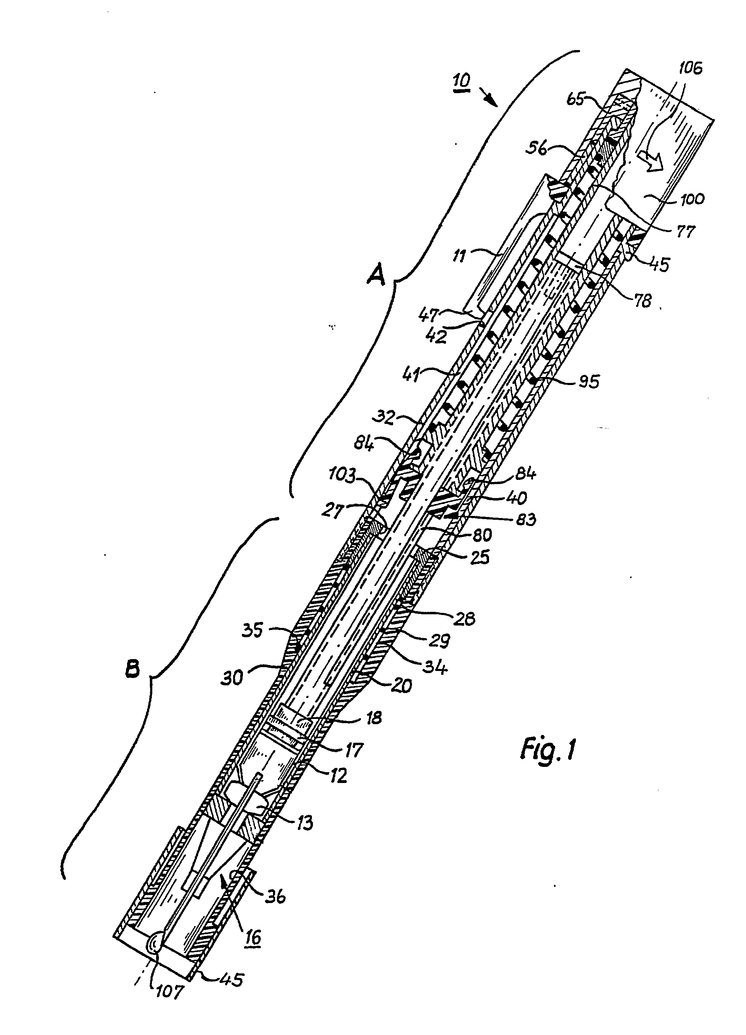

Fig. 1 zeigt ein erfindungsgemäßes Injektionsgerät 10 im zusammengeschobenen Zustand, den es in der Ruhelage und nach einer Injektion einnimmt. Dieses Gerät hat etwa die Form eines überdimensionierten Füllfederhalters und hat seitlich einen Befestigungsclip 11 der von Füllfederhaltern bekannten Form; dieser Clip 11 dient als Auslöseglied für die Injektion.

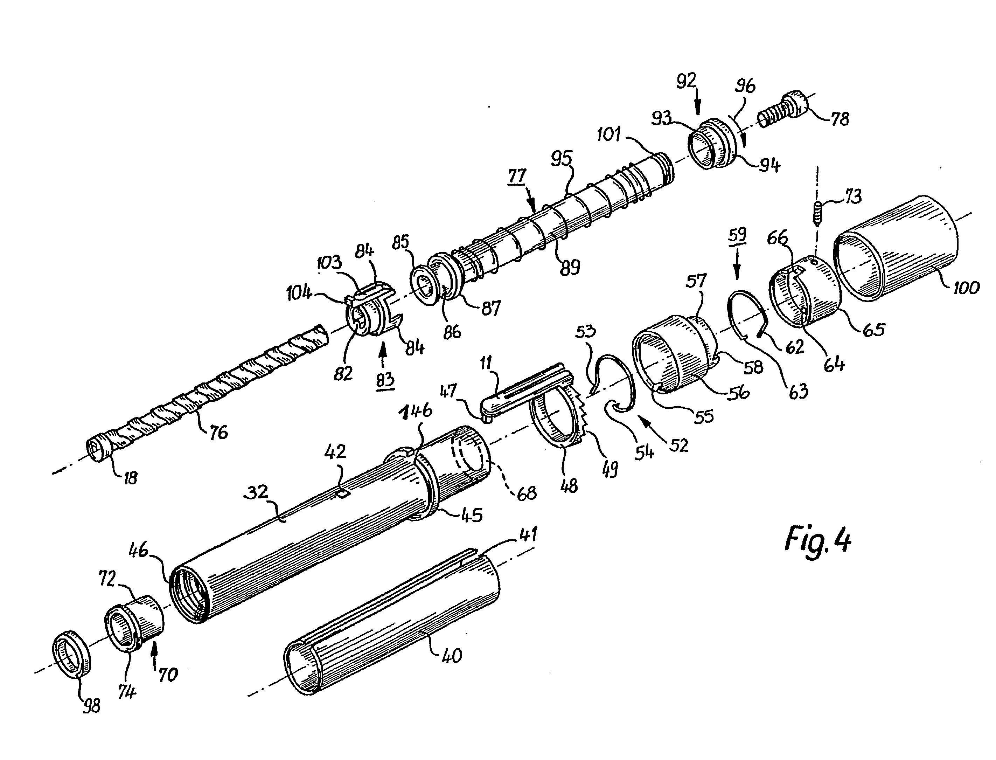

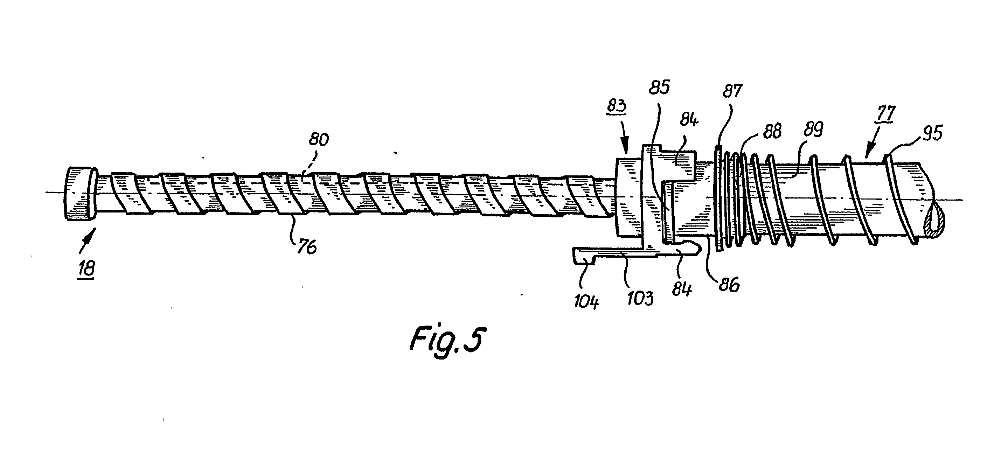

Das Injektionsgerät 10 hat einen Teil A, der zur Einstellung der zu injizierenden Flüssigkeitsmenge sowie zur Durchführung der Injektion dient und der in Fig. 4 in auseinandergezogener, raumbildlicher Darstellung gezeigt ist, um das Verständnis zu erleichtern. Außerdem zeigt Fig. 5 ein wichtiges Element des Teils A in raumbildlicher Darstellung.

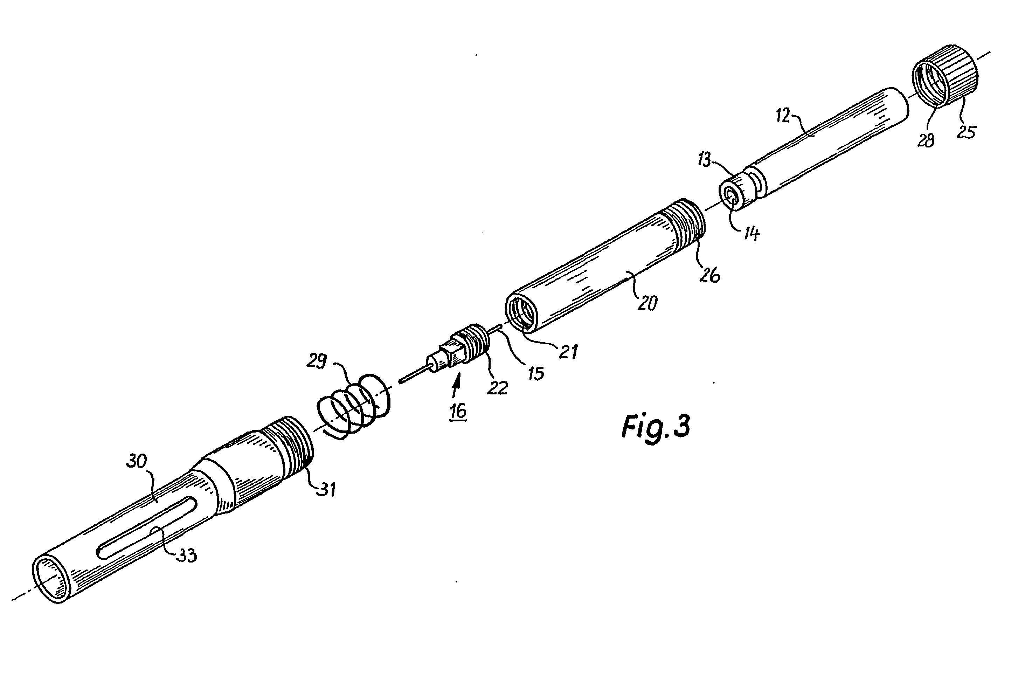

Ferner hat das Injektionsgerät 10 einen Teil B, der zur Aufnahme eines Behälters 12, auch Kartusche genannt, mit der zu injizierenden Flüssigkeit dient. Dieser Teil B ist in Fig. 3 in auseinandergezogener, raumbildlicher Darstellung gezeigt, um hier ebenfalls das Verständnis zu erleichtern.

Wie man den Fig. 1 und 3 entnimmt, hat der Behälter 12 die Form eines länglichen, zylindrischen Glasröhrchens, das sich am proximalen, also dem Patienten zugewandten Ende verjüngt und dort mit einer aufgebördelten Aluminiumkappe 13 versehen ist, in der sich eine dünne Gummimembran 14 befindet, die vom distalen Ende 15 einer mit 16 bezeichneten Injektionsnadel, auch Kanüle genannt, durchstoßen werden kann . Derartige Behälter mit Insulin werden z.B. von der Firma Novo Industri AB, Dänemark, unter der Marke Penfill vertrieben. In dem Glasröhrchen 12 befindet sich ein verschiebbarer Kolben 17, der z.B. aus einem geeigneten Gummi hergestellt sein kann und der unter der Wirkung eines Stößels 18 (Fig. 1, 2, 4 und 5) in proximaler Richtung verschoben werden kann, um eine Injektion zu bewirken.

Zur Aufnahme des Behälters 12 dient eine Aufnahmehülse 20, die an ihrem proximalen Ende mit einem Innengewinde 21 versehen ist, das zur Aufnahme eines entsprechenden Außengewindes 22 der Injektionsnadel 16 dient. Da nämlich der Behälter 12 Flüssigkeit für mehrere Injektionen erhält, z.B. 100 Einheiten Insulin, muß die Nadel 16 nach jeder Injektion gegen eine neue, sterile Nadel ausgetauscht werden, und dies geschieht durch Einschrauben in das Gewinde 21, wobei die Membran 14 jeweils nach dem Herausziehen des distalen Nadelendes 15 wieder einen dichten Verschluß bildet.

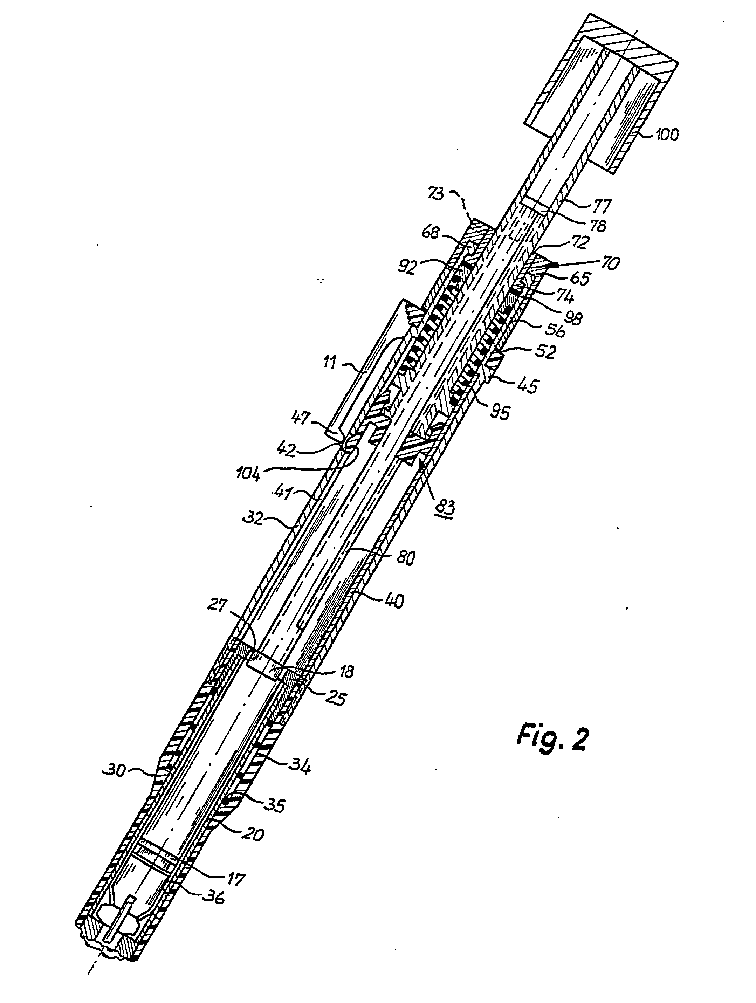

Zum Verschließen des distalen Endes der Außenhülse 20 dient eine außen gerändelte Überwurfkappe 25, die auf ein Außengewinde 26 am distalen Ende der Aufnahmehülse 20 aufgeschraubt werden kann. Diese Überwurfkappe 25 ist mit einer zentralen Ausnehmung 27 (Fig. 1 und 2) versehen, durch welche der Stößel 18 unbehindert durchdringen kann. Die Überwurfkappe 25 bildet an ihrem proximalen Ende eine Schulter 28, welche als Widerlager für eine Rückstellfeder 29 dient. Die Aufnahmehülse 20 wird zweckmäßig aus einem durchsichtigen Kunststoff hergestellt, um eine visuelle Beobachtung des Behälters 12 zu ermöglichen und um erkennen zu können, wie groß der Flüssigkeitsvorrat in diesem noch ist.

Ein wichtiges Merkmal der vorliegenden Erfindung ist, daß die Aufnahmehülse 20 sehr genau an die Behälter 12 der einzelnen Hersteller angepaßt werden kann und dadurch verhütet, daß irrtümlich ein falscher Behälter mit einer höheren Dosierung verwendet wird, was beim Patienten z.B. zu einer Überdosierung mit Insulin mit den bekannten schädlichen Folgen (Koma) führen könnte.

Zur verschiebbaren Aufnahme der Aufnahmehülse 20 dient das vordere (proximale) Gehäuseteil 30, das an seinem distalen Ende mit einem Außengewinde 31 zur Verbindung mit dem hinteren (distalen) Gehäuseteil 32 versehen ist und ein Fenster 33 hat, durch welches der Inhalt des Behälters 12 beobachtet werden kann. Das vordere Gehäuseteil 30 kann aus Metall, z.B. Aluminium, oder aus einem geeigneten Kunststoff, z.B. Polypropylen, hergestellt werden. Wie die Figuren 1 und 2 zeigen, hat das Gehäuseteil 30 in seinem distalen Bereich einen zylindrischen Hohlraum 34 größeren Durchmessers, der über eine Ringschulter 35 in einen zylindrischen Hohlraum 36 kleineren Durchmessers übergeht. Der Durchmesser des Hohlraums 36 ist dabei nur geringfügig größer als der Außendurchmesser der zylindrischen Aufnahmehülse 20, so daß letztere im Hohlraum 36 axial verschoben werden kann. Die Feder 29 liegt im montierten Zustand zwischen der Ringschulter 28 an der Überwurfkappe 25 und der Ringschulter 35 und beaufschlagt folglich die Aufnahmehülse 20 in distaler Richtung. In der Lage des Geräts direkt nach einer Injektion ist die Feder 29 gespannt, d.h. die Injektionsnadel 16 ist, im Vergleich zu Fig. 1, in proximaler Richtung weiter nach vorne geschoben, und der Stößel 18 liegt mit Vorspannung gegen den Kolben 17 an und verhindert, daß sich die Feder 29 entspannt. Erst vor der nächsten Injektion, wenn der Stößel 18 zurückgezogen ist, wie das Fig. 2 zeigt, entspannt sich die Feder 29 und drückt die Aufnahmehülse 20 in distaler Richtung bis zum Anschlag gegen eine im hinteren Gehäuseteil 32 befestigte zylindrische Hülse 40 (Fig. 4). Die Hülse 40 hat einen Längsschlitz 41, der zur Längsführung des Stößels 18 dient und der mit einer fensterartigen Öffnung 42 im hinteren Gehäuseteil 32 fluchtet.

Bei der Montage des B-Teils wird zunächst ein neuer Behälter 12 in der in Fig. 3 dargestellten Lage in die Aufnahmehülse 20 eingeschoben, und die Überwurfkappe 25 wird dann auf das Außengewinde 26 aufgeschraubt. Anschließend wird mittels einer (nicht dargestellten) Hilfsvorrichtung, welche die Injektionsnadel 16 steril umgibt, diese Nadel mit ihrem Außengewinde 22 in das Innengewinde 21 der Aufnahmehülse 20 eingeschraubt, wobei das distale Nadelende 15 die Membran 14 durchstößt und in die Flüssigkeit im Behälter 12 eindringt. Anschließend wird die Aufnahmehülse 20 durch das distale Ende in das vordere Gehäuseteil 30 eingeführt, in dem sich bereits die Feder 29 befindet. Zum Schutz der Injektionsnadel 16 kann dieses Gehäuseteil 30 an seinem proximalen Ende mit einer Schutzhülse 45 (Fig. 1) oder mit einer Schutzkappe versehen sein.

Nachdem das vordere Gehäuseteil 30 so geladen worden ist, kann es mit seinem Gewinde 31 in ein entsprechendes Gewinde 46 des hinteren Gehäuseteils 32 eingeschraubt werden und ist dann z.B. für mehrere Injektionen nach der sogenannten intensivierten Insulintherapie bereit, d.h. aus dem Behälter 12 können - je nach dem Insulinbedarf des Patienten - mehrere Injektionen mit vorgewählter Dosierung erfolgen. Wie dies geschieht, ergibt sich aus der nachfolgenden Beschreibung des A-Teils.

Hierbei soll auch noch erwähnt werden, daß der Außendurchmesser der Überwurfkappe 25 kleiner als der Innendurchmesser der zylindrischen Ausnehmung 34 gewählt ist, so daß die aus der Aufnahmehülse 20, der Injektionsnadel 16 und der Überwurfkappe 25 gebildete Einheit entgegen der Kraft der Rückstellfeder 29 im vorderen Gehäuseteil 30 in proximaler Richtung so weit verschiebbar ist, bis die Feder 29 zusammengepreßt ist. Eine solche Verschiebung erfolgt beim Injektionsvorgang, wobei dann die Injektionsnadel 16 aus dem vorderen Gehäuseteil 30 heraustritt und in das Gewebe des Patienten einsticht.

Im hinteren Gehäuseteil 32 ist, wie bereits beschrieben, die Hülse 40 befestigt, deren Längsschlitz 41 mit dem Fenster 42 des Gehäuseteils 32 fluchtet, d.h. durch dieses Fenster 42 kann man in das Innere der Hülse 40 schauen.

Auf der Außenseite des Gehäuseteils 32 und nahe bei dessen distalem Ende ist ein Ring 45 befestigt; er hat eine Unterbrechung 146, die auf derselben Mantellinie des Gehäuseteils 32 liegt wie das Fenster 42. Die Unterbrechung 146 dient zur Fixierung des Clips 11, der mit einem nach innen ragenden Vorsprung 47 versehen ist, welcher im montierten Zustand dem Fenster 42 gegenüberliegt, vgl. Fig. 1 und 2.

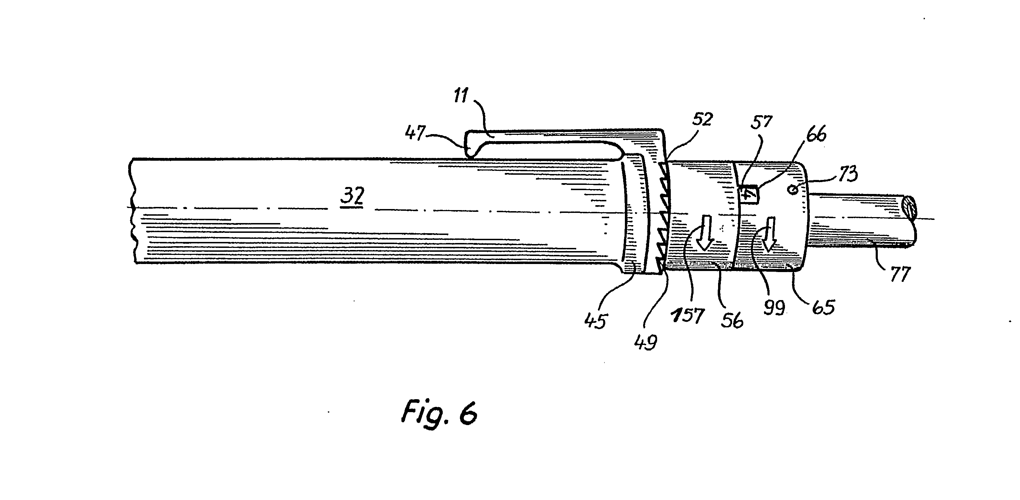

Der Clip 11, der z.B. als Spritzgußteil aus Polyamid ausgebildet sein kann, hat einen Haltering 48, welcher auf seiner distalen Seite mit sägezahnartigen Rastzähnen 49 versehen ist. Schaut man auf das distale, also in Fig. 1 obere Ende des Injektionsgeräts, so sperren diese Zähne gegen eine Drehung im Uhrzeigersinn, wie sich das auch aus den Fig. 4 und 6 ohne weiteres ergibt.

Der Haltering 48 wird auf das distale Ende des hinteren Gehäuseteils 32 so weit aufgeschoben, bis er satt gegen den Ring 45 anliegt, wobei der Clip 11 durch die Unterbrechung 46 hindurchragt und durch diese gegen Drehung gesichert ist.

Auf den Haltering 48 folgt in distaler Richtung ein Federring 52, der an seinem in Fig. 4 linken Ende mit einem in proximaler Richtung abgebogenen Rastabschnitt 53 zum Rasteingriff in die Rastzähne 49 versehen ist. Außerdem hat dieser Federring 52 an seinem in Fig. 4 rechten Ende einen rechtwinklig in distaler Richtung abgebogenen Abschnitt 54 zum Eingriff in eine entsprechende Ausnehmung 55 eines Vorwählrads 56. Auf diese Weise kann das Vorwählrad, das auf dem distalen Ende des Gehäuseteils 32 drehbar gelagert ist, nur in der in Fig. 6 mit einem Pfeil 157 angegebenen Richtung verdreht werden.

Das Vorwählrad 56 hat einen Abschnitt 57 reduzierten Durchmessers, und auf diesem befindet sich eine Einstellskala, z.B. in Form von Zahlen, in Fig. 6 symbolisch angedeutet durch die arabische Ziffer "4".

In der distalen Stirnseite des Abschnitts 57 befindet sich eine sägezahnförmige Vertiefung 58, deren Form sich aus Fig. 4 ergibt. Zum Eingriff in sie dient ein Federring 59, und zwar dessen in Fig. 4 rechtes Ende 62, das in proximaler Richtung schräg abgebogen ist. Sein in Fig. 4 linkes Ende 63 ist in distaler Richtung rechtwinklig abgebogen und greift in ein axiales Loch 64 eines Stellglieds 65, welches mit einem Fenster 66 zur Anzeige der Skala (auf dem Abschnitt 57) versehen ist und das auf dem Abschnitt 57 drehbar geführt ist.

Das Gehäuseteil 32 hat an seinem distalen Ende einen radial nach innen ragenden Kragen 68, und dieser bildet einen Anschlag für eine erste Bundhülse 70. Diese hat einen zylindrischen Abschnitt 72, welcher durch die Öffnung am distalen Ende des Gehäuseteils 32 hindurchragt und auf dessen distalem Ende das Stellglied 65 mittels einer Madenschraube 73 befestigt ist. Ferner hat die Bundhülse 70 einen radial ab stehenden Bund 74, der, wie dargestellt, gegen die proximale Seite des Kragens 68 anliegt und dadurch das Stellglied 65 drehbar am distalen Ende des Gehäuseteils 32 lagert.

Der Stößel 18 hat auf seiner Außenseite ein Steilgewinde 76, das in Fig. 5 in raumbildlicher Darstellung gezeigt ist und das in einem entsprechenden Innengewinde (nicht dargestellt) einer rohrförmig ausgebildeten Verlängerungsanordnung 77 geführt ist. Verdreht man also Stößel 18 und Verlängerungsanordnung 77 relativ zueinander, so ändert sich die Gesamtlänge dieser Kombination in der einen oder der anderen Richtung.

Der Stößel 18 wird in die Verlängerungsanordnung 77 eingeschraubt und dort durch eine Konterschraube 78 gesichert, welche in ein Gewinde am distalen Ende des Stößels 18 eingeschraubt wird. Der Stößel 18 kann dann nur noch bis zu einem - durch die Schraube 78 gebildeten - Anschlag aus der Verlängerungsanordnung 77 herausgeschraubt werden. Fig. 5 zeigt diese Maximalstellung.

Der Stößel 18 hat eine Längsnut 80, die sich, wie dargestellt, bis in die Nähe seines proximalen Endes erstreckt und in die ein radial nach innen ragender Vorsprung 82 eines Führungsglieds 83 ragt.

Das Führungsglied 83 hat im Rahmen des erfindungsgemäßen Geräts mehrere Funktionen. Es ist auf seiner distalen Seite mit drei krallenartigen Vorsprüngen 84 versehen, welche über einen Ringbund 85 am proximalen Ende der Verlängerungsanordnung 77 hinübergreifen und hinter diesem Ringbund eingerastet sind, so daß das Führungsglied 83 mit der Verlängerungsanordnung 77 frei drehbar verbunden ist, aber axiale Kräfte auf die Verlängerungsanordnung bzw. von dieser übertragen kann. Da das Führungsglied 83 zweckmäßig aus einem elastischen Kunststoff, z.B. einem Polyamid, ausgebildet ist, können die krallenartigen Vorsprünge 84 auf den Ringbund 85 aufgeclipst werden, so daß die Montage sehr einfach ist.

Anschließend an den Ringbund 85 hat die Verlängerungsanordnung eine Ringnut 86, auf die wiederum ein Ringbund 87 folgt, der über einen Abschnitt 88 größeren Durchmessers in einen Abschnitt 89 kleineren Durchmessers übergeht. Eine zweite Bundhülse 92 ist axial verschiebbar auf dem Abschnitt 89 der Verlängerungsanordnung 77 angeordnet, wobei ihr zylindrischer Abschnitt 93 in proximaler Richtung und ihr radial nach außen ragender Bund 94 in distaler Richtung angeordnet ist.

Eine Schlingfeder 95 ist auf der Verlängerungsanordnung 77 angeordnet und umgibt den Abschnitt 89 kleineren Durchmessers lose. Ihr proximales Ende ist eng auf den Abschnitt 88 größeren Durchmessers aufgeschoben, und ebenso ist ihr distaler Abschnitt eng auf den zylindrischen Abschnitt der zweiten Bundhülse 92 aufgeschoben.

Da der Drehsinn der Schlingfeder 95 der Darstellung in Fig. 4 und 5 entspricht, kuppelt sie die zweite Bundhülse 92 mit der Verlängerungsanordnung 77, wenn die zweite Bundhülse 92 in Richtung des in Fig. 4 dargestellten Pfeiles 96 verdreht wird. (Blickt man auf das distale Ende des Injektionsgeräts, so entspricht dies einer Drehung der zweiten Bundhülse 92 entgegen dem Uhrzeigersinn.) Wird dagegen die zweite Bundhülse 92 entgegen der Richtung des Pfeiles 96 verdreht, so öffnet sich die Schlingfeder 95, und die zweite Bundhülse 92 kann sich drehen, ohne die Verlängerungsanordnung 77 ebenfalls zu verdrehen. Es ist hier auch darauf hinzuweisen, daß die Schlingfeder 95 im Rahmen der Erfindung ebenfalls eine Doppelfunktion hat, nämlich einmal als Federanordnung zur Betätigung des Stößels 18, und zum anderen als Schlingfederkupplung. Auch dies stellt ein bedeutsames Merkmal der Erfindung dar.

Im zusammengebauten Zustand liegt zwischen der zweiten Bundhülse 92 und der ersten Bundhülse 70 ein Kupplungsorgan 98, z.B. ein O-Ring, der als Rutschkupplung wirkt. Das Kupplungsorgan 98 ermöglicht es, eine Verdrehung des Stellglieds 65 über die erste Bundhülse 70, das Kupplungsorgan 98, die zweite Bundhülse 92 und die Schlingfeder 95 auf die Verlängerungsanordnung 77 zu übertragen, wenn diese Bewegung in Richtung des Pfeiles 99 (Fig. 6) entspricht dem Pfeil 96 der Fig. 4 erfolgt. Ist dabei der Stößel 18 bis zu seiner maximalen Länge ausgedreht, so daß die als Anschlag dienende Schraube 78 wirksam wird, so tritt das Kupplungsorgan 98 in Aktion und wirkt als Rutschkupplung, d.h. die zweite Bundhülse 92 steht, während die erste Bundhülse 70 weiterhin vom Benutzer verdreht werden kann.

Am distalen Ende der Verlängerungsanordnung 77 ist ein Handgriff 100 dadurch befestigt, daß er auf ein Außengewinde 101 (Fig. 4) der Verlängerungsanordnung 77 aufgeschraubt ist. Zieht man in distaler Richtung an diesem Handgriff 100, so wird die Feder 95 zusammengepreßt und dadurch gespannt, da sich der Ringbund 87 der Verlängerungsanordnung 77 in Richtung zur zweiten Bundhülse 92 bewegt. Dabei wird auch - über die krallenartigen Vorsprünge 84 - das Führungsglied 83 in distaler Richtung mitgezogen.

Das Führungsglied 83 hat auf seiner Außenseite ein federndes Rastglied 103, welches in der Längsnut 41 der Hülse 40 axial geführt ist und dadurch eine Verdrehung des Führungsgliedes 83 und des in ihm axial geführten Stößels 18 verhindert. An seinem freien Ende hat das Rastglied 103 eine Rastnase 104, und diese rastet in das Fenster 42 ein, wenn die Verlängerungsanordnung 77 durch den Handgriff 100 genügend weit in distaler Richtung gezogen wird. Diese eingerastete Stellung ist in Fig. 2 dargestellt, und in dieser Stellung ist die Feder 95 gespannt. Drückt nun der Benutzer auf den als Auslöseglied dienenden Clip 11, so drückt dessen Vorsprung 42 gegen die Rastnase 104, drückt diese radial nach innen und gibt das Führungsglied 83 frei, so daß dieses, der Stößel 18 und die Verlängerungsanordnung 77 eine Bewegung in proximaler Richtung ausführen, wobei sich die Feder 95 wieder weitgehend entspannt.

Arbeitsweise

Der B-Teil des Injektionsgeräts wird, wie das weiter oben beschrieben wurde, mit einem Behälter 12 mit dem gewünschten Medikament geladen.

Anschließend wird durch Drehen des - hier als Stellglied dienenden - Handgriffs 100 in Richtung des Pfeiles 106 (Fig. 1) die Verlängerungsanordnung 77 so verdreht, daß der Stößel 18 in proximaler Richtung bewegt wird und den Kolben 17 im Behälter 12 in Injektionsrichtung bewegt. Dabei bildet sich an der Spitze der Injektionsnadel 16 ein Tropfen 107, welcher dem Benutzer anzeigt, daß der Stößel 18 richtig gegen den Kolben 17 anliegt. Die Hülse 20 und die Nadel 16 sind hierbei, wie bereits erwähnt, im Vergleich zu Fig. 1 stärker in proximaler Richtung verschoben, und die Feder 29 ist entsprechend zusammengepreßt.

Nun wird der Handgriff 100 in distaler Richtung gezogen und die Feder 95 wird so weit gespannt, bis die Rastnase 104 in das Fenster 42 einrastet. Dabei gibt der Handgriff 100 die Einstellvorrichtung frei. Die Hülse 20 verschiebt sich hierbei unter der Wirkung der Feder 29 in die in Fig. 2 dargestellte Lage.

Das Vorwählrad 56 (Fig. 6) wird nun so weit in Richtung des Pfeiles 57 verdreht, bis im Fenster 66 die gewünschte Dosierung angezeigt wird, z.B., wie in Fig. 6 dargestellt, vier Einheiten. Dabei gleitet die Ringfeder 52 über die Zähne 49 und verursacht bei jedem Zahn ein klickendes Geräusch, so daß auch ein blinder oder sehschwacher Patient durch Zählen der Klickgeräusche die richtige Dosierung einstellen kann. Bei dieser Einstellung wird das Stellglied 65 über die Schlingfeder 95 und das Kupplungsorgan 98 festgehalten, dreht sich also nicht mit. Dabei dreht sich folglich das Ende 62 der Feder 59 aus der Vertiefung 58 heraus.

Anschließend an diesen Vorwahlvorgang dreht der Benutzer das Stellglied 65 in Richtung des Pfeiles 99 (Fig. 6), was, wie bereits beschrieben, über das Kupplungsorgan 98 und die Schlingfeder 95 eine entsprechende Verdrehung der Verlängerungsanordnung 77 und einen entsprechenden Vorschub des Stößels 18 bewirkt, d.h. die Gesamtlänge von Verlängerungsanordnung 77 und Stößel 18 wird entsprechend der gewählten Dosierung größer.

Diese Bewegung des Stellglieds 65 in Richtung des Pfeiles 99 wird dadurch begrenzt, daß der Vorsprung 62 der Feder 59 wieder in die Vertiefung 58 gelangt und eine weitere Bewegung des Stellglieds 65 sperrt. Wurde z.B. zuvor das Vorwählrad 56 um 40° verdreht, so kann das Stellglied nur um ebenfalls 40° nachgestellt werden und kann entsprechend die Verlängerungsanordnung 77 um 40° verdrehen. Das Injektionsgerät ist nun bereit, denn der Patient hat seine Dosis, z.B. an Insulin, eingestellt. Er setzt nun das Injektionsgerät auf den Körperteil auf, an dem die Injektion stattfinden soll, und drückt dann auf den Clip 11.

Dadurch wird das Führungsglied 83 freigegeben, und mit ihm der Stößel 18, so daß dieser sich mit erheblicher Geschwindigkeit in proximaler Richtung bewegt und dabei auf den Kolben 17 trifft. Da die Flüssigkeit im Behälter 12 einer sofortigen Bewegung des Kolbens 17 im Behälter 12 widersteht, wird der gesamte Behälter 12 einschließlich der Führungshülse 20 entgegen der Kraft der (schwachen) Feder 29 in proximaler Richtung bewegt, wodurch die Nadel 16 in das Gewebe des Patienten einsticht. Anschließend bewegt sich dann der Kolben 17 unter der Wirkung des Stößels 18 ebenfalls in proximaler Richtung und bewirkt die Injektion der vorgewählten Flüssigkeitsmenge in den Patienten.

Da die Injektion nur in einer einzigen Richtung erfolgt, ist sie für den Patienten weitgehend schmerzfrei. Außerdem kann sie mit nur einer Hand erfolgen, so daß mit einem erfindungsgemäßen Gerät z.B. auch am Rücken und am Gesäß injiziert werden kann, also größere Flächen für Injektionen zur Verfügung stehen. Dies ist sehr wichtig bei der intensivierten Insulintherapie mit ihrer wesentlich höheren Zahl von Injektionen. Naturgemäß kann die Einstellung des Injektionsgeräts auch dadurch erfolgen, daß der Handgriff 100 in Richtung des Pfeiles 106 entsprechend weit verdreht wird, wobei dann zweckmäßig zwischen dem Gehäuseteil 32 und dem Handgriff eine entsprechende, ebenfalls nachstellbare Skala vorgesehen wird. Für die Einstellung eines erfindungsgemäßen Injektionsgeräts gibt es, wie ohne weiteres ersichtlich, viele Möglichkeiten und Varianten, so daß die dargestellte Version - die besonders für blinde Patienten geeignet ist - nur als eine unter mehreren Möglichkeiten zu betrachten ist.

In der vorstehenden Beschreibung wurde hauptsächlich auf die Injektion von Insulin Bezug genommen. Ein erfindungsgemäßes Injektionsgerät kann aber naturgemäß in der gleichen Weise auch für die Injektion anderer Substanzen verwendet werden, z.B. für die Injektion von Hydroxycobalamin bei Anämie, die Injektion von schmerzstillenden Mitteln bei Krebskranken, oder bei Tierärzten für die Reihenimpfung von mehreren Tieren.

**Patentansprüche**

1. Injektionsgerät zur dosierten Einspritzung, mit einer Aufnahmevorrichtung (20) für einen insbesondere als Kartusche ausgebildeten Behälter (12), welcher die zu injizierende Flüssigkeit enthält, mit einer Injektionsnadel (16), die beim Injektionsvorgang in proximaler Richtung relativ zum Gehäuse (30, 32) des Injektionsgeräts bewegbar ist, und mit einem Stößel (18), welchem eine Federanordnung (95) zugeordnet ist, welche letztere vom Benutzer spannbar und nach dem Spannen durch Betätigen einer Auslösevorrichtung auslösbar ist, um eine Bewegung des Stößels (18) in proximaler Richtung und dadurch eine Bewegung der Injektionsnade (16) in proximaler Richtung sowie ein Auspressen von zu injizierender Flüssigkeit aus dem Behälter (12) und damit eine Injektion von in dem Behälter (12) befindlicher Flüssigkeit zu bewirken, ferner mit einer dem Stößel (18) zugeordneten Verlängerungsanordnung (77), welche mit diesem verstellbar in der Weise verbunden ist, daß die wirksame Gesamtlänge von Stößel (18) und Verlängerungsanordnung (77) mittels einer Stellvorrichtung (56, 65; 100) änderbar und dadurch die Menge der beim Einspritzvorgang zu injizierenden Flüssigkeit dosierbar ist, dadurch gekennezicht, daß der Stößel (18) mit einem Gewinder (76) versehen ist, welches mit einem Gewinde der Verlängerungsanordnung (77) in Eingriff steht, daß die Stellvorrichtung (56, 65; 100) zum Erzeugen einer Drehbewegung zwischen Verlängerungsanordnung (77) und Stößel (18) ausgebildet ist, und daß der Stößel (18) im Gehäuse (30, 32) des Injektionsgeräts längsverschiebbar, aber gegen Drehung relativ zu diesem Gehäuse gesichert, angeordnet ist.

2. Injektionsgerät nach Anspruch 1, dadurch gekennzeichnet, daß der Stößel (18) eine Längsnut (80) aufweist, in welche ein im Gehäuse (32) längsverschiebbares, aber relativ zum Gehäuse nicht verdrehbares Führungsglied (83) eingreift.

3. Injektionsgerät nach Anspruch 2, dadurch gekennzeichnet, daß im Gehäuse (32) ein Längsführungsorgan, insbesondere in Form einer Längsnut (41), vorgesehen ist, welches mit dem Führungsglied (83) in Eingriff steht und dieses in Längsrichtung führt.

4. Injektionsgerät nach Anspruch 2 oder 3, dadurch gekennzeichnet, daß das Führungsglied (83) mit der Verlängerungsanordnung (77) verbunden, aber relativ zu ihr verdrehbar ist, und daß es zum Übertragen einer axialen Kraft auf die Verlängerungsanordnung (77) oder von dieser ausgebildet ist.

5. Injektionsgerät nach mindestens einem der Ansprüche 1 bis 4, dadurch gekennzeichnet, daß das Gewinder des Stößels (18) als Außengewinde (76), insbesondere als Steilgewinde, ausgebildet ist.

6. Injektionsgerät nach mindestens einem der vorhergehenden Ansprüche, dadurch gekennzeichnet, daß die Verlängerungsanordnung (77) direkt mit einem ihre Verdrehung ermöglichenden Stellglied (100) verbunden ist.

7. Injektionsgerät nach Anspruch 6, dadurch gekennzeichnet, daß das Stellglied (100) als Handgriff zum Spannen einer dem Stößel (18) zugeordneten Federanordnung (95) ausgebildet ist.

8. Injektionsgerät nach mindestens einem der vorhergehenden Ansprüche, dadurch gekennzeichnet, daß die Verlängerungsanordnung (77) über eine Kupplung (98) insbesondere eine Rutschkupplung, eine Freilaufkupplung, oder eine Schlingefederkupplung, mit einem ihre Verdrehung ermöglichenden Stellglied (65) verbunde ist.

9. Injektionsgerät nach mindestens einem der vorhergehenden Ansprüche, dadurch gekennzeichnet, daß die dem Stößel (18) zugeordnete Federanordnung (95) auf die Verlängerungsanordnung (77) wirkt.

10. Injektionsgerät nach mindestens einem der vorhergehenden Ansprüche, dadurch gekennzeichnet, daß die Auslösevorrichtung eine auf die Kombination von Stößel (18) und Verlängerungsanordnung (77) wirkende Sperre (42, 104) aufweist, welche diese Kombination nach dem Spannen in ihrer gespannten Lage hält, und daß die Auslösevorrichtung einen Auslöser zum Lösen dieser Sperre (42, 104) aufweist, welcher vorzugsweise als Clip (11) ausgebildet ist.

11. Injektionsgerät nach Anspruch 10, dadurch gekennzeichnet, daß die Sperre ein mit der Kombination von Stößel (18) und Verlängerungsanordnung (77) in Wirkverbindung stehendes Rastglied (103) aufweist, welches eine Rastnase (104) zum Eingriff in eine gehäusefeste Ausnehmung (42) des Injektionsgeräts aufweist, wobei die Rastnase (104) vorzugsweise in einer gehäusefesten Längsnut (41) des Injektionsgeräts geführt ist, und wobei vorzugsweise das Rastglied (103) am Führungsglied (83) ausgebildet ist.

12. Injektionsgerät nach Anspruch 11, dadurch gekennzeichnet, daß das Rastglied (103) die Längsführung des Führungsglieds (83) im Gehäuse (32) bewirkt.

13. Injektionsgerät nach mindestens einem der vorhergehenden Ansprüche, dadurch gekennzeichnet, daß die Stellvorrichtung (56, 65) mit einer Ratsche, oder mit Rastzähnen (43), oder mit einer anderen beim Verstellen ein hörbares, auch einem Blinden eine Dosierung ermöglichendes Geräusch erzeugenden Einrichtung versehen ist, und daß sie vorzugsweise mit einer Anzeige (57) für die gewählte Dosierung versehen ist.

14. Injektionsgerät nach mindestens einem der vorhergehenden Ansprüche, dadurch gekennzeichnet, daß die Stellvorrichtung ein Vorwählrad (56) aufweist, welches auf eine vorgewählte Dosierung einstellbar ist,
und daß diesem Vorwählrad (56) ein Stellglied (65) zugeordnet ist, welches nach Vorwahl der Dosierung bis zu einem durch die Vorwahl verstellten Anschlag (58) verdrehbar ist und dabei die Gesamtlänge von Stößel (18) und Verlängerungsanordnung (77) entsprechend der gewählten Dosierung vergrößert.

**Claims**

1. Injection device for dosed injection, with a holding device (20) for a container (12) in particular having the form of a cartridge and containing the liquid to be injected, with a hypodermic needle (16) which can be moved in the proximal direction relative to the housing (30, 32, of the injection device during the injection operation( and with a plunger (18) which is associated with a spring arrangement (95) which can be cocked by the user and after cocking can be released by actuating a triggering device in order to move the plunger (18) in the proximal direction and thereby move the hypodermic needle (16) in the proximal direction and force liquid to be injected out of the container (12) and so inject liquid held in the container (12), furthermore with an extension arrangement (77) which is associated with the plunger (18) and is connected with the latter in an adjustable manner so that the effective total length of the plunger (18) and the extension arrangement (77) can be altered by means of a setting device (56, 65; 100) and thus the quantity of the liquid to be injected during the injection operation can be dosed, characterised in that the plunger (18) is provided with a thread (76) which engages with a thread of the extension arrangement (77), in that the setting device (56, 65; 100) is designed to produce rotation between the extension arrangement (77) and the plunger (18), and in that the plunger (18) is disposed in the housing (30, 32) of the injection device so that it can be moved longitudinally but is prevented from rotating relative to this housing.

2. Injection device according to claim 1, characterised in that the plunger (18) comprises a longitudinal channel (80) in which engages a guiding element (83) which can be moved longitudinally in the housing (32) but cannot rotate relative to the housing.

3. Injection device according to claim 2, characterised in that a longitudinal guiding element. in particular in the form of a longitudinal slot (41), which engages with the guiding element (83) and guides this in the longitudinal direction, is provided in the housing (32).

4. Injection device. according to claim 2 or 3, characterised in that the guiding element (83) is connected with the extension arrangement (77) but can rotate relative to it, and in that it is designed to transmit an axial force to or from the extension arrangement (77).

5. Injection device according to at least one of claims 1 to 4, characterised in that the thread of the plunger (81) takes the form of an external thread (76), in particular a coarse thread.

6. Injection device according to at least one of the preceding claims, characterised in that the extension arrangement (77) is connected directly with a setting element (100) permitting its rotation.

7. Injection device according to claim 6, characterised in that the setting element (100) takes the form of a handle for cocking a spring arrangement (95) associated with the plunger (18).

8. Injection device according to at least one of the preceding claims, characterised in that the extension arrangement (77) is connected by means of a coupling (98), in particular a slipping coupling, a one-way coupling or a gripping spring coupling, with a setting element (65) permitting its rotation.

9. Injection device according to at least one of the preceding claims, characterised in that the spring arrangement (95) associated with the plunger (18) acts on the extension arrangement (77).

10. Injection device according to at least one of the preceding claims, characterised in that the triggering device comprises a lock (42, 104) which acts on the combination of the plunger (18) and the extension arrangement (77) and, after cocking, holds this combination in its cocked position, and in that the triggering device comprises a trigger to release this lock (42, 104), which trigger preferably takes the form of a clip (11).

11. Injection device according to claim 10, characterised in that the lock comprises a locking element (103) which is operatively connected to the combination of the plunger (18) and the extension arrangement (77) and comprises a locking lug (104, for engagement in an opening (42) fixed in the housing of the injection device, and the locking lug (104) is preferably guided in a longitudinal slot (41) fixed in the housing of the injection device and preferably the locking element (103) is formed on the guiding element (83).

12. Injection device according to claim 11, characterised in that the locking element (103) is adapted to guide the guiding element (83) longitudinally in the housing (32).

13. Injection device according to at least one of the preceding claims, characterised in that the setting device (56, 65) is provided with a ratchet or with locking teeth (43) or with another device producing an audible noise during setting so that a blind person can also set the dosage, and in that it is preferably provided with an indicator (57) for the selected dosage.

14. Injection device according to at least one of the preceding claims, characterised in that the setting device comprises a pre-selector wheel (56) which can be set to a pre-selected dosage, and in that this pre-selector wheel (56) is associated with a setting element (65) which after pre-selection of the dosage can be rotated as far as a stop (58) set by the pre-selection, thereby increasing the total length of the plunger (18) and the extension arrangement (77) in accordance with the selected dosage.

**Revendications**

1. Dispositif d'injection destiné à une injection dosée, comprenant un dispositif (20) de réception destiné à un réservoir (12) constitué en particulier comme une cartouche, et qui contient le fluide à injecter, une aiguille d'injection (16), qui peut se déplacer en direction proximale par rapport au boîtier (30, 32) du dispositif d'injection pendant le processus d'injection et, un poussoir (18), auquel est associé un agencement de ressort (95), ce dernier pouvant être sollicité par l'utilisateur, puis être relâché après mise en sollicitation, par actionnement d'un dispositif de déclenchement, pour effectuer un déplacement du poussoir (18) en direction proximale et de cette façon un déplacement de l'aiguille d'injection (16) dans la direction proximale ainsi qu'une éjection de fluide à injecter hors du réservoir (12) et, ainsi, une injection de fluide se trouvatn dans le réservoir (12), ainsi qu'un dispositif d'allongement (77) associé au poussoir (18), lié à ce dernier de façon réglable et de façon telle que la longeur totale acitve du poussoir (18) et du dispositif d'allongement (77) peut être modifiée à l'aide d'un dispositif de réglage (56, 65;

100) et que, de cette façon la quantité de fluide à injecter lors du processus d'injection est dosable, caractérisé en ce que le poussoir (18) comporte un filetage (76), qui est en engagement avec un filetage du dispositif d'allongement (77), en ce que le dispositif de réglage (56, 65; 100) est constitué pour créer un déplacement de rotation entre le dispositif d'allongement (77) et le poussoir (18), et en ce que le poussoir (18) est agencé de façln à être mobile longitudinalement dans le boîtier (30, 32) du dispositif d'injection, mais ne peut tourner par rapport à ce boîtier.

2. Dispositif d'injection selon la revendication 1, caractérisé en ce que le poussoir (18) présente une rainure longitudinale (80), dans laquelle pénètre un organe de guidage (83) mobile longitudinalement dans le boîtier (32) mais sans pouvoir tourner par rapport au boîtier.

3. Dispositif d'injection selon la revendication 2, caractérisé en ce que le boîtier (32) comprend un organe de guidage longitudinal, et particulier sous la forme d'une rainure longitudinale (41), qui est en engagement avec l'organe de guidage (83) et qui guide celui-ci dans la direction longitudinale.

4. Dispositif d'injection selon la revendication 2 ou 3, caractérisé en ce que l'organe de guidage (83) est lié au dispositif d'allongment (77), mais peut tourner par rapport à celui-ci, et en ce qu'il est constitué pour transférer und force axiale sur le dispositif d'allongement (77) ou depuis celui-ci.

5. Dispositif d'injection selon l'une au moins des revendications 1 à 4, caractérisé en ce que le filetage du poussoir (18) est constitué comme un filetage extérieur (76), en particulier un filetage à pas rapide.

6. Dispositif d'injection selon l'une au moins des revendications précédentes, caractérisé en c que le dispositif d'allongment (77) est directement lié à un organe de réglage (100) qui permet sa rotation.

7. Dispositif d'injection selon la revendication 6, caractérisé en ce que le dispositif de réglage (100) est constitué comme une poignée pour solliciter un dispositif à ressor (95) associé au poussoir (18).

8. Dispositif d'injection selon l'une au moins des revendications précédentes, caractérisé en ce que le dispositif d'allongement (77) est lié à un organe de réglage (65) permettant sa rotation, par l'intermédiaire d'un accouplement (98), en particulier d'un accouplement à friction, d'un accouplement à roue libre ou d'un accouplement par ressort en spirale.

9. Dispositif d'injection selon l'une au moins des revendications précdédentes, caractérisé en ce que le dispositif à ressort (95) associé au possoir (18) agit sur le dispositif d'allongement (77).

10. Dispositif d'injection selon l'une au moins des revendications précédentes, caractérisé en ce que le dispositif de déclenchement présente un dispositif de blocage (42, 104) qui agit sur la combinaison poussoir (18), dispositif d'allongement (77) et qui maintient cette combinaison dans sa position sollicitée après la mise en sollicitation, et en ce que le dispositif de déclenchement présente une gâchette pour libérer ce dispositif de blocage (42, 104), qui est de préférence constitué comme une attache formant ressort (11).

11. Dispositif d'injection selon la revendication 10, caractérisé en ce que le dispositif de blocage présente un organe d'encliquetage (103) qui est en liaison active avec la combinaison du poussoir (18) et du dispositif d'allongement (77), qui présente un nez d'encliquetage (104) destiné à s'engager dans un évidement (42) fixe par rapport au boîtier, du dispositif d'injection, le nez d'encliquetag (104) étant de préférence guidé dans une rainure longitudinale (41) fixe par rapport au boîtier du dispositif d'injection, et l'organe d'encliquetage (103) étant de préférence formé sur l'organe de guidage (83).

12. Dispositif d'injection selon la revendication 11, caractérisé en ce que l'organe d'encliquetage (103) effectue le guidage longitudinal de l'organe (83) de guidage dans le boîtier (32).

13. Dispositif d'injection selon l'une au moins des revendications précédentes, caractérisé en ce que le dispositif de réglage (56, 65) comporte un cliquet, ou des dents (43) qui peuvent être encliquetées ou un autre dispositif qui émet, lors d'un déplacment, un bruit audible, permettant un dosage même par aveugle, et en ce qu'il comporte de préférence un indicatuer (57) du dosage choisi.

14. Dispositif d'injection selon l'une au moins des revendications précédentes, caractérisé en ce que le dispositif de réglage présente une roue de présélection (56), qui peut être réglée pour un dosage présélectionné, et en ce qu'un organe de réglage (65) est associé à cette roue de pré-sélection et peut tourner après la pré-sélection du dosage jusqu'à une butée (58) déplacée par le pré-sélection, et allonge ainsi, d'une façon correspondant au dosage choisi, la longueur totale du poussoir (18) et du dispositif d'allongement (77).

*Fig. 1*

Fig. 2

Fig.3

EP 0 268 191 B1

Fig.4

EP 0 268 191 B1

Fig.5

Fig. 6